# EUROPEAN PATENT SPECIFICATION

(11) **EP 3 479 856 B1**
(45) Date of publication and mention of the grant of the patent: **19.08.2026**
(21) Application number: 17820131.5
(22) Date of filing: 27.06.2017
(51) Int. Cl.: A61M 5/00, A61M 5/14, A61M 5/19, A61M 5/31, A61M 5/145, A61M 5/315, A61M 5/36, A61M 39/00, A61M 39/10, A61J 1/20, B01F 23/451, B01F 25/10, B01F 25/433, B01F 33/501

(54) **MIXING DEVICE, MIXING TUBE, INJECTION SYSTEM, AND METHOD FOR PRODUCING MIXING DEVICE**
MISCHVORRICHTUNG, MISCHROHR, EINSPRITZSYSTEM UND VERFAHREN ZUR HERSTELLUNG EINER MISCHVORRICHTUNG
DISPOSITIF DE MÉLANGE, TUBE DE MÉLANGE, SYSTÈME D'INJECTION ET PROCÉDÉ DE PRODUCTION D'UN DISPOSITIF DE MÉLANGE

(30) Priority: 30.06.2016 JP 2016130356
(43) Date of publication of application: 08.05.2019
(73) Proprietor: Nemoto Kyorindo Co., Ltd., Tokyo 113-0033 (JP)
(72) Inventor: WATANABE Takashi, Tokyo 113-0033 (JP); KAWABE Tomoyuki, Tokyo 113-0033 (JP)
(74) Representative: Grünecker Patent- und Rechtsanwälte PartG mbB
(86) International application number: PCT/JP2017/023509
(87) International publication number: WO 2018/003777

(56) References cited:
- WO-A1-2011/125303
- WO-A1-2014/168210
- JP-A- 2011 217 796
- US-A1- 2006 089 603
- US-A1- 2014 261 713

## Description

### Technical Field

The present invention relates to a mixing device for mixing a plurality of kinds of liquid medicines, a mixing tube including the mixing device, an injection system including the mixing device, and a manufacturing method of the mixing device.

### Background Art

As medical fluoroscopic imaging apparatuses, there are imaging apparatuses such as a MRI (Magnetic Resonance Imaging) apparatus, a CT (Computed Tomography) apparatus, an angiography imaging apparatus, a PET (Positron Emission Tomography) apparatus, a SPECT (Single Photon Emission Computed Tomography) apparatus, a CT angiography apparatus, a MR angiography apparatus, an ultrasonic diagnostic apparatus, and a vascular imaging apparatus. When imaging a subject with these imaging apparatuses, a plurality of kinds of liquid medicines (for example, a contrast agent and a physiological saline solution) having different specific gravities or viscosities may be mixed and injected into the subject's body in order to obtain a clear image.

As a device for mixing liquid medicines, for example, a mixing device described in Patent Literature 1 is known. This mixing device includes a swirling flow generating chamber for generating a swirling flow, a first flow inlet for introducing a contrast agent into the swirling flow generating chamber, a second inlet for introducing a physiological saline solution into the swirling flow generating chamber, and a narrowing chamber provided between an outlet from which a mixed liquid medicine flows out and the swirling flow generating chamber. A further example of a turbulent mixing chamber is known from Patent Literature 2.

### Citation List

### Patent Literature

PTL 1: Japanese Patent Application Laid-Open No. 2011-217796 ; and PTL 2: United States Patent Application Publication No. US 2014/0261713.

### Summary of Invention

### Technical Problem

The following method can be considered as a method of manually venting the air from a mixing tube including the mixing device described in Patent Literature 1. First, a first syringe for contrast agent is connected to the first inlet via the mixing tube, and a second syringe for physiological saline solution is connected to the second inlet via the mixing tube. Next, a piston of the first syringe is pushed up with the piston facing down in the gravity direction, and a channel from the first syringe to the mixing device is filled with the contrast agent. Then, a piston of the second syringe is pushed up with the piston facing down in the gravity direction, and a channel from the second syringe to the mixing device and a channel from the mixing device to a tip of the mixing tube are filled with the physiological saline solution.

However, since air bubbles are likely to be trapped in the first inlet side corner of the mixing device, in order to vent the air, it was necessary to use a lot of physiological saline solution. Additionally, a method of venting the air by simultaneously pushing out the physiological saline solution and the contrast agent is also conceivable. However, there was a problem that an expensive contrast agent is consumed. Furthermore, it is generally desirably to improve the mixing efficiency of mixing devices.

### Solution to Problem

In order to solve the above problems, a mixing device and manufacturing method as respectively defined in appended claims 1 and 9 is provided. Further embodiments are given in the dependent claims.

Additionally, a mixing tube as another example of the present invention is characterized by including a tube configured to communicate with the second inlet, a tube configured to communicate with the outlet, and the above mixing device.

Additionally, an injection system as another example of the present invention is characterized by including an injection apparatus to which a first syringe filled with the first liquid medicine and a second syringe filled with the second liquid medicine are mounted, and the above mixing tube connected to the first syringe and the second syringe.

### Advantageous Effects of Invention

In this manner, when a user manually vents the air, it is possible to suppress that air bubbles remain in the mixing device, and to vent the air with a small amount of liquid medicine.

The further features of the present invention will become apparent from the following description of examples illustratively shown with reference to the accompanying drawings.

### Brief Description of Drawings

FIG. 1 is a schematic perspective view of an entire injection system.
FIG. 2 is a schematic side view of a mixing tube.
FIG. 3 is a schematic side view of a mixing device according to a first embodiment.
FIG. 4 is a schematic cross-sectional view along the longitudinal direction of the mixing device.
FIG. 5 is a schematic cross-sectional view of an enlarged jointing portion.
FIG. 6 is an explanatory diagram for describing a joining process.
FIG. 7 is a schematic side view of a mixing device according to a second embodiment.

### Description of Embodiments

Hereinafter, exemplary embodiments for practicing the present invention are described in detail with reference to the drawings. However, the sizes, the materials, the shapes, and the relative positions of components and the like that are described in the following embodiments are arbitrary, and can be changed according to the configurations or various conditions of an apparatus to which the present invention is applied. Additionally, unless otherwise described, the scope of the present invention is not limited to the embodiments specifically described below. Further, in this specification, up and down correspond to the upward direction and the downward direction in the gravity direction, respectively.

### [First embodiment]

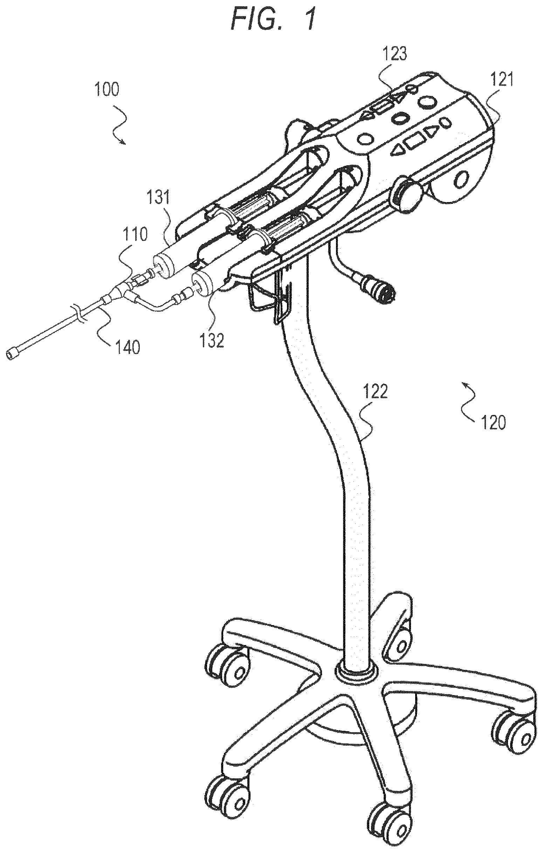

FIG. 1 is a schematic perspective view of an injection system 100 for liquid medicine including a mixing device 110 according to the present invention. As shown in FIG. 1, the injection system 100 for liquid medicine for injecting liquid medicines such as a contrast agent into a subject includes an injection apparatus (injector) 120 mounted with a first syringe 131 filled with a first liquid medicine (for example, a contrast agent), and a second syringe 132 filled with a second liquid medicine (for example, a physiological saline solution). Additionally, the Injection system 100 includes a mixing tube 140 connected to the first syringe 131 and the second syringe 132. Additionally, the injection system 100 includes the first syringe 131 and the second syringe 132 that are installed in an injection apparatus 120. Further, the mixing tube 140 is connected to a conduit portion at the tip of the first syringe 131 and a conduit portion at the tip of the second syringe 132.

The injection apparatus 120 includes an injection head 121 on which the first syringe 131 and the second syringe 132 are mounted, and a control apparatus (not shown) controlling the injection head 121. Further, the injection head 121 is wiredly connected to the control apparatus via a metal cable and an optical cable. This control apparatus and the injection head 121 may be wirelessly connected to each other, and may be connected with a radio system using a frequency band of 2.4 GHz to 5 GHz, for example. Additionally, a remote operation apparatus, such as a hand switch, may be wiredly or wirelessly connected to the injection head 121 or the control apparatus. Further, the power supply of the injection head 121 can be provided in the injection head 121 or the control apparatus. Additionally, the power supply independent of the injection head 121 can also be provided separately, and the power supply can also be replaced with a battery.

The injection head 121 is mounted on a movable caster stand 122 placed on a floor surface, and is rotatably held in the upper part of the caster stand 122. In this manner, the injection head 121 can be rotated to a posture in which the tip side of the injection head 121 (the side on which the first syringe 131 and the second syringe 132 are installed) is oriented to the floor surface, and a posture in which the rear end side of injection head 121 (the side on which the first syringe 131 and the second syringe 132 are not installed) is oriented to the floor surface. Further, a ceiling hanging member can be provided instead of the caster stand 122, and the injection head 121 can also be hanged from a ceiling via this ceiling-hanging member.

Additionally, the injection head 121 is provided with a plurality of operation buttons 123, such as a move forward button, an acceleration button, a move backward button, a check button, and a start button. Further, a user can operate the operation buttons 123 to manually operate the injection head 121. Specifically, while the user is pressing the move forward button, a pressing portion of the injection head 121 moves forward, and while the user is pressing the move backward button, the pressing portion moves backward. Additionally, when the user presses the check button, the injection head 121 stands by in an injectable state. Then, when the user presses the start button, the injection head 121 moves the pressing portion forward and starts injection of a liquid medicine. Additionally, since the operation buttons are also displayed on a touch panel of the control apparatus, the user can also operate the touch panel to cause the injection head 121 to perform injection.

Additionally, the injection head 121 includes a drive mechanism that is not shown. For example, the drive mechanism includes a transmission mechanism connected to a shaft of a motor, a screwed shaft connected to the transmission mechanism, a feed screw nut attached to the screwed shaft, and an actuator connected to the feed screw nut. The transmission mechanism includes a pinion gear connected to the shaft, and a screw gear connected to the screwed shaft. Further, the transmission mechanism transmits the rotation from the motor to the screwed shaft. In this manner, the rotation of the shaft of the motor is transmitted to the screwed shaft via the pinion gear and the screw gear. Therefore, the screwed shaft rotates according to the transmitted rotation, and the feed screw nut slides in a move forward direction or a move backward direction with the rotation of the screwed shaft. With the sliding of this feed screw nut, the pressing portion moves forward or moves backward.

The injection head 121 is provided with two syringe receiving portions, and the first syringe 131 and the second syringe 132 are mounted in the syringe receiving portions. The first syringe 131 is filled with a first liquid medicine having a large specific gravity (for example, a contrast agent), and the second syringe 132 is filled with a second liquid medicine (for example, a physiological saline solution) having a specific gravity smaller than the first liquid medicine. As specific examples of the contrast agent, there are a contrast agent with an iodine concentration of 240 mg/mL (for example, viscosity 3.3 Pa·s, and specific gravity 1.268 to 1.296 at 37 °C), a contrast agent with an iodine concentration of 300mg/mL (for example, viscosity 6.1 mPa·s, specific gravity 1.335 to 1.371 at 37 °C), and a contrast agent with an iodine concentration of 350mg/mL (for example, viscosity 10.6 mPa·s, specific gravity 1.392 to 1.433 at 37 °C). Additionally, as specific examples of the physiological saline solution, there is a physiological saline solution containing 180 mg of sodium chloride in 20 mL of the physiological saline solution (for example, viscosity 0.9595 mPa·s, specific gravity 1.004 to 1.006 at 20 °C).

In each of the first syringe 131 and the second syringe 132, a piston slidable in the syringe is attached. Further, when the motor rotates normally in the state where the rear end of the piston contacts the pressing portion, the pressing portion will press the piston in the move forward direction. When the piston moves forward, the liquid medicine in the syringe is pushed out, and is injected into a subject's body via the mixing tube connected to the tip of the syringe. On the other hand, when the motor is reversed, the pressing portion will draw the piston in the retreat direction.

These first syringe 131 and second syringe 132 may be either of a syringe filled with a liquid medicine and an empty syringe not filled with a liquid medicine. The syringe filled with a liquid medicine includes a prefilled syringe filled with a liquid medicine in advance, a syringe obtained by manually filling an empty syringe with a liquid medicine by the user, and a syringe obtained by filling an empty syringe with a liquid medicine by the user by using a suction instrument or a filling instrument. Additionally, when an empty syringe is mounted to the injection head 121, the user can fill the syringe with a liquid medicine with the injection head 121, the suction instrument, or the filling instrument.

Additionally, a data carrier, such as a RFID (Radio Frequency Identifier) or a bar code, can be provided in the first syringe 131 and the second syringe 132. For example, the information of the filled liquid medicine is recorded on this data carrier. Further, the injection apparatus 120 can read the recorded information from the data carrier via the injection head 121, and can control the injection pressure of the liquid medicine. For example, the control apparatus can calculate an optimum injection amount per weight based on the read information of the liquid medicine, (the amount of iodine, the gadolinium content, or the like), and can display the optimum injection amount on the touch panel of the control apparatus.

### [Mixing Tube]

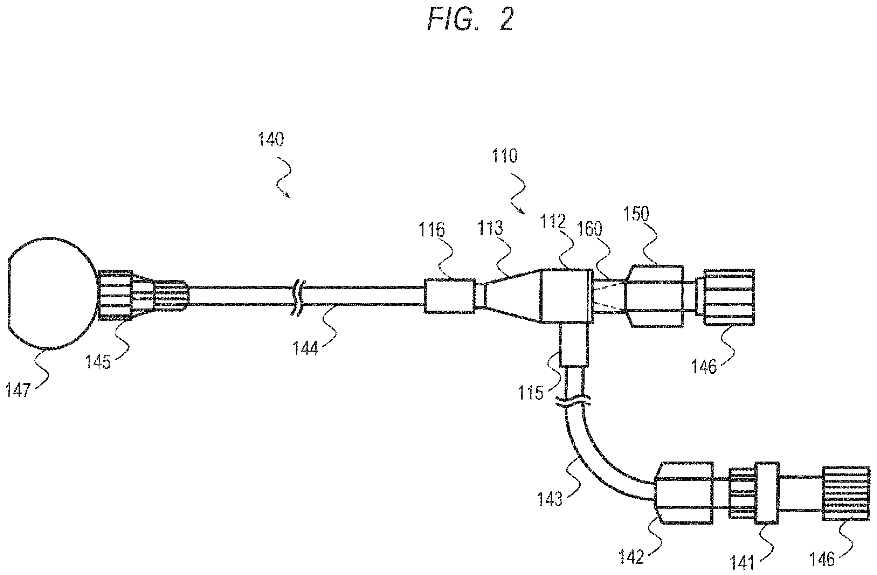

FIG. 2 is a schematic side view along the longitudinal direction of the mixing tube 140 including the mixing device 110. As shown in FIG. 2, the mixing tube 140 includes a flexible tube 143 communicating with a second inlet of the mixing device 110, a flexible tube 144 communicating with an outlet of the mixing device 110, and the mixing device 110 connected to the first syringe 131 via a holding portion 150.

Additionally, the mixing tube 140 includes unidirectional valve 141 connected to the second syringe 132. This unidirectional valve prevents a mixed liquid medicine from flowing backward to the second syringe 132. Additionally, the mixing tube 140 includes a female connector 142 connected to the unidirectional valve 141. The tube 143 is joined to this female connector 142 and a conduit portion 115 of the mixing device 110. Specifically, the downstream end portion of the tube 143 in the flowing direction of the liquid medicine is inserted into the inside of the conduit portion 115, and joined to the inner surface of the conduit portion 115. Each of the holding portion 150, the unidirectional valve 141, and the female connector 142 is connected by a method, such as screwing, solvent adhesion, or adhesion by an ultraviolet curing type adhesive.

Additionally, the mixing tube 140 includes the tube 144 connected to a conduit portion 116 of the mixing device 110. This tube 144 is inserted into the inside of the conduit portion 116, and joined to the inner surface of the conduit portion 116. Additionally, the mixing tube 140 includes a male rotary lock connector 145 connected to the tip of the tube 144. A removable liquid reservoir cap 147 is attached to the male rotary lock connector 145. The liquid reservoir cap 147 is used at the time of venting the air from the mixing tube 140, and is removed at the time of injection. Additionally, removable caps 146 are attached to the unidirectional valve 141 and the holding portion 150 of mixing device 110. The caps 146 are removed at the time of injection of the liquid medicine of the mixing tube 140.

For example, the mixing device 110, the female connector 142, and the male rotary lock connector 145 can be formed from polycarbonate. Additionally, the caps 146 can be formed from polyethylene, the unidirectional valve 141 can be formed from polycarbonate and silicone rubber, the tubes 143 and 144 can be formed from polyvinyl chloride, and the liquid reservoir cap 147 can be formed from polypropylene. Further, the tubes 143 and 144 may be rigid tubes.

The contrast agent pushed out from the first syringe 131 flows into a swirling flow generating chamber 112 via the holding portion 150 of the mixing device 110. Additionally, the physiological saline solution pushed out from the second syringe 132 flows into the swirling flow generating chamber 112 via the unidirectional valve 141, the female connector 142, and the tube 143. Then, the contrast agent and the physiological saline solution are mixed in the swirling flow generating chamber 112 and a narrowing chamber 113. Thereafter, the mixed liquid medicine of the contrast agent and the physiological saline solution flows out to the tube 144 via the conduit portion 116.

### [Mixing Device]

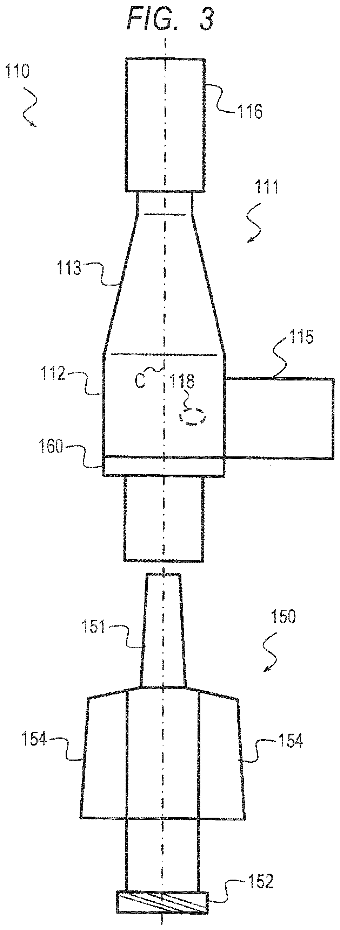

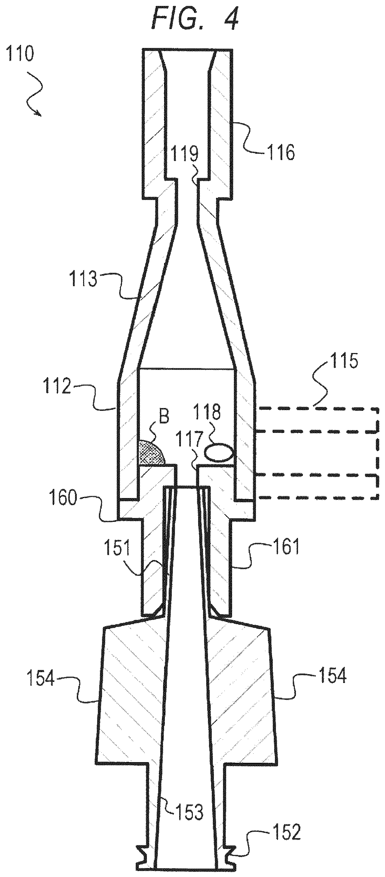

FIG. 3 is a schematic side view along the longitudinal direction of the mixing device 110, and FIG. 4 is a schematic cross-sectional view along the longitudinal direction of the middle of the mixing device 110. Further, in FIG. 3, for convenience of description, the holding portion 150 before joining is shown separated from the swirling flow generating chamber 112. Additionally, in FIG. 4, a conduit portion 115, which does not appear in the cross section, is indicated by a dotted line.

As shown in FIGS. 3 and 4, the mixing device 110 includes the swirling flow generating chamber 112 for generating a swirling flow, a first inlet 117 (FIG. 4) for introducing the first liquid medicine into the swirling flow generating chamber 112, a second inlet 118 for introducing the second liquid medicine into the swirling flow generating chamber 112, an outlet 119 (FIG. 4) from which the mixed liquid medicine of the first liquid medicine and the second liquid medicine flows out, and a narrowing chamber 113 provided between the swirling flow generating chamber 112 and the outlet 119, and having a space continuously narrowed toward the outlet 119.

Additionally, the mixing device 110 includes the conduit portion 115 that extends from a side surface of the swirling flow generating chamber 112, and to which the tube 143 is connected, and the conduit portion 116 that is located in the downstream side of the narrowing chamber 113 in the flowing direction of the mixed liquid medicine, and to which the tube 144 is joined. Further, the mixing device 110 has a tip portion 151 extending in a direction parallel to a center line C of the swirling flow generating chamber 112, and includes the holding portion 150 holding the swirling flow generating chamber 112.

The swirling flow generating chamber 112 has a substantially cylindrical outer shape, and has a curved inner surface forming a substantially columnar internal space for concentrating a swirling flow in an axis direction. Additionally, the swirling flow generating chamber 112 communicates with the narrowing chamber 113. Further, the swirling flow generating chamber 112 has a jointing portion 160 to be joined with the tip portion 151 of the holding portion 150. This jointing portion 160 configures an upstream side end face in the flowing direction of the liquid medicine, and the first inlet 117 (FIG. 4) is formed in the jointing portion 160. Additionally, the second inlet 118 to be communicated with the tube 143 inserted into the conduit portion 115 is formed in the swirling flow generating chamber 112. Further, the cross-sectional shape of the swirling flow generating chamber 112 in the transverse direction may be formed by a circle, an ellipse, or other curved lines.

The narrowing chamber 113 is provided between the swirling flow generating chamber 112 and the outlet 119 (FIG. 4), and is continuously narrowed toward the outlet 119 in an axially symmetric manner. That is, the narrowing chamber 113 has a substantially funnel-shaped outer shape, and a substantially circular-truncated-cone internal space that is coaxial with the internal space of the swirling flow generating chamber 112. Additionally, the narrowing chamber 113 communicates with the outlet 119. Further, the narrowing chamber 113 includes an inclined inner surface extending toward the outlet 119 so that the funnel-shaped space is formed. Accordingly, the swirling flow generated in the swirling flow generating chamber 112 is concentrated in the central axis direction of vortex. Further, the internal shape of the narrowing chamber 113 may be a shape that narrows toward the conduit portion 116, and may be formed by a gradual stair-like inner surface.

The conduit portion 115 has a substantially cylindrical shape, and communicates with the second inlet 118 of the swirling flow generating chamber 112. Therefore, the conduit portion 115 is provided in a curved side surface outside of the swirling flow generating chamber 112, and extends in the tangential direction of the circumference of the swirling flow generating chamber 112. In other words, the conduit portion 115 is provided at a position shifted from the central axis line of the columnar space that the swirling flow generating chamber 112 includes to the periphery side of the swirling flow generating chamber 112. In this manner, the swirling flow of the liquid medicine flowed from the conduit portion 115 is generated within the swirling flow generating chamber 112. The second inlet 118 is opened with a shape curved along the shape of the curved inner surface of the swirling flow generating chamber 112.

The conduit portion 116 has a substantially cylindrical shape, and the center line of the conduit portion 116 matches the center line of the first inlet 117 (FIG. 4). That is, the first inlet 117, the swirling flow generating chamber 112, the narrowing chamber 113, the outlet 119, and the conduit portion 116 are coaxial. In this manner, since each of the constituent portions are arranged to have a common axis, the isotropy of the vortex generated in the mixing device 110 can be improved. That is, the vortex can be smoothly and uniformly generated within the space, and the mixing efficiency can be improved.

The rigid holding portion 150 holds the swirling flow generating chamber 112 when the mixing device 110 is stood so that the conduit portion 116 is oriented upward. Additionally, the holding portion 150 includes the tip portion 151 extending along the longitudinal direction (the direction parallel to the center line C of the swirling flow generating chamber 112) of the mixing device 110. This tip portion 151 is inserted into a receiving portion 161 (FIG. 4) of the jointing portion 160. The size of the tip portion 151 is smaller than the receiving portion 161, and the tip portion 151 has a tapered shape. Additionally, the holding portion 150 includes a terminal end portion 152 to which the first syringe 131 filled with the first liquid medicine is to be detachably connected. This terminal end portion 152 has a thread groove, and the mixing device 110 is connected to the first syringe 131 via the holding portion 150.

Additionally, the holding portion 150 includes an internal flow passage 153 (FIG. 4) extending to the tip portion 151 from the terminal end portion 152, and a pair of wing-like portions 154 protruding to both sides and interposing the internal flow passage 153 between the wing-like portions 154. This internal flow passage 153 has a tapered shape in which the cross-sectional area is decreased from the terminal end portion 152 to the tip portion 151 so that the liquid medicine (contrast agent) flows through the internal flow passage 153. That is, the internal flow passage 153 is formed to be continuously narrowed toward the first inlet 117 from the opening on the side of the terminal end portion 152. Further, the diameter of a downstream side exit of the internal flow passage 153 in the flowing direction of the liquid medicine is set to be substantially the same as the diameter of the first inlet 117. In this manner, it is possible to suppress deceleration of the flow of the liquid medicine flowing into the first inlet 117, compared with the case where there is a level difference between the tip portion 151 and the first inlet 117.

The jointing portion 160 of the swirling flow generating chamber 112 includes the substantially cylindrical receiving portion 161 that receives the tip portion 151 of the holding portion 150. The tip portion 151 inserted into the jointing portion 160 communicates with the swirling flow generating chamber 112 via the first inlet 117. In this manner, the liquid medicine having a large specific gravity that passed through the first inlet 117 is introduced into the swirling flow generating chamber 112 in a direction parallel to the central axis of the swirling flow of the liquid medicine having a small specific gravity. That is, the liquid medicine having a large specific gravity is introduced in the direction parallel to the central axis line of the columnar space that the swirling flow generating chamber 112 has.

### [Manufacturing Method of Mixing Device]

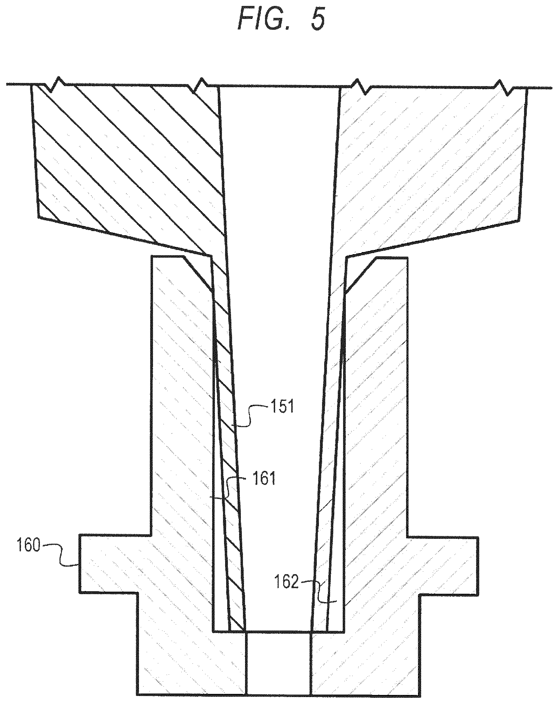

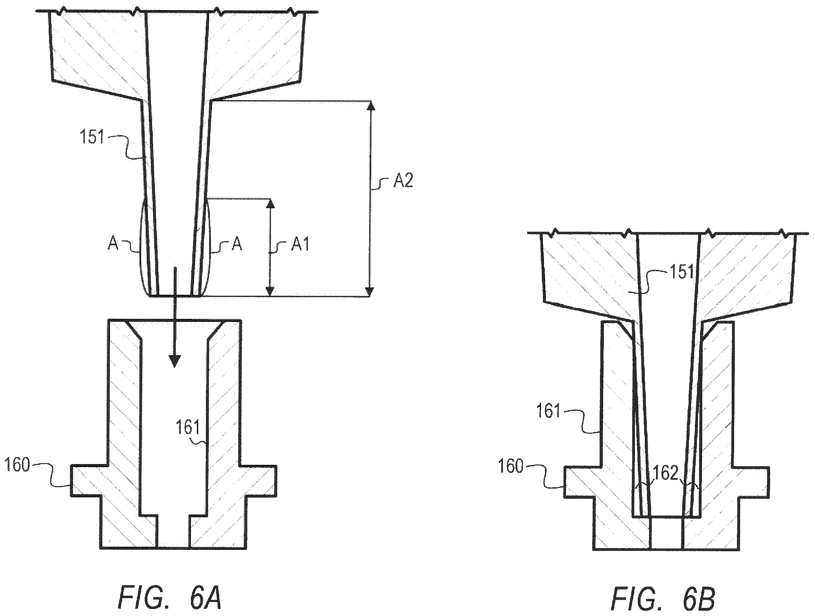

A manufacturing method of the mixing device 110 is described with reference to FIG. 5 showing a schematic enlarged cross-section of the joining part of the jointing portion 160 and the holding portion 150, and FIGS. 6A and 6B showing a joining process.

First, a body 111 (FIG. 3) of the mixing device 110, the jointing portion 160, and the holding portion 150 are prepared. For example, the body 111, the jointing portion 160, and the holding portion 150 can be formed by molding resin, such as polycarbonate. On this occasion, the body 111 is formed to include the swirling flow generating chamber 112, the narrowing chamber 113, the conduit portion 115, and the conduit portion 116. Additionally, the holding portion 150 is formed to have the tip portion 151 smaller than the receiving portion 161 of the jointing portion 160. That is, the holding portion 150 is formed so that the outer diameter of the tip portion 151 becomes smaller than the inner diameter of the receiving portion 161. More preferably, the holding portion 150 is formed so that the tip portion 151 has a tapered shape.

The holding portion 150 is formed, and at the same time, the swirling flow generating chamber 112 is formed to have the jointing portion 160 including the receiving portion 161. Specifically, an ultraviolet curing type adhesive is applied to the body 111 and/or the jointing portion 160, and the body 111 and the jointing portion 160 are bonded together. Thereafter, both of them are fixed by irradiating ultraviolet light to the joining part, and the swirling flow generating chamber 112 is formed. Further, the body 111 and the jointing portion 160 may be bonded together by solvent adhesion or ultrasonic welding. Additionally, a thread groove and a screw thread may be formed in the body 111 and the jointing portion 160, respectively, and both of them may be joined by screwing.

Next, an adhesive is applied between the tip portion 151 and the jointing portion 160. Specifically, as shown in FIG. 6A, an ultraviolet curing type adhesive A is applied to at least at the end portion of the tip portion 151 of the holding portion 150, more preferably, to a plurality of places or over the whole circumference of an area A1 from the end portion to the middle. Additionally, the tip portion 151 is inserted into the receiving portion 161 of the jointing portion 160 until an end surface of the tip portion 151 contacts a bottom surface (a step provided between the receiving portion 161 and the first inlet 117) of the receiving portion 161. In this manner, an entire area A2 of the tip portion 151 is inserted into the receiving portion 161. When the tip portion 151 is inserted into the receiving portion 161, a gap (an adhesive reservoir) 162 resulting from the size difference is formed (FIG. 6B) between the tip portion 151 and the receiving portion 161. The gap 162 provided between the tip portion 151 and the receiving portion 161 widens from the vicinity of the root of the tip portion 151 toward its farthest portion. Additionally, the amount of application of the adhesive A is set in advance to be accumulated in the gap 162. Further, since the tip portion 151 contacts the bottom surface of the receiving portion 161, the adhesive A does not flow into the first inlet 117.

When the tip portion 151 is fully inserted, a circumferential surface in the vicinity of the root of the tip portion 151 contacts the inner surface of the receiving portion 161. In this manner, the tip portion 151 is positioned within the receiving portion 161, and the exit of the internal flow passage 153 communicates with the first inlet 117. Further, the diameter of the exit of the internal flow passage 153 may be formed to be slightly larger than the diameter of the first inlet 117. In this manner, even if the tip portion 151 is deformed by contacting the receiving portion 161 and thus the internal flow passage 153 is narrowed, the form of the exit of the internal flow passage 153 can be substantially matched with the form of the first inlet 117.

Thereafter, ultraviolet light is irradiated to the joining part of the tip portion 151 and the receiving portion 161, so that the adhesive A is cured and the holding portion 150 is fixed to the jointing portion 160. With the ultraviolet light irradiation, the adhesive accumulated in the gap 162 is cured, and the joining force of the tip portion 151 and the receiving portion 161 can be increased. In this manner, the mixing device 110 is formed. Further, the adhesive A may be applied after the tip portion 151 is inserted into the receiving portion 161. For example, after the tip portion 151 is inserted into the receiving portion 161, the adhesive A may be applied to the whole circumference or about four places of the root of the tip portion 151 using an application machine, and the adhesive A may be poured into the gap 162 using capillarity. Additionally, the tip portion 151 may be inserted into the receiving portion 161 after applying the adhesive A to the bottom surface of the receiving portion 161.

### [Mixing of Liquid Medicines]

When mixing liquid medicines, the liquid medicine (the contrast agent) having a large specific gravity from the first syringe 131 flows through the holding portion 150 and the jointing portion 160 and flows into the swirling flow generating chamber 112 from the first inlet 117. When the contrast agent flows into the swirling flow generating chamber 112, the contrast agent becomes a jet flow. Then, the momentum of the jet flow is diffused in the outer periphery direction of the swirling flow generating chamber 112. That is, the flow of a contrast agent is diffused in the outer periphery direction of the swirling flow generating chamber 112.

Additionally, the liquid medicine (the physiological saline solution) having a small specific gravity from the second syringe 132 flows through the conduit portion 115 and flows into the swirling flow generating chamber 112 from the second inlet 118. The physiological saline solution is guided from the second inlet 118 to a curved inner surface of the swirling flow generating chamber 112, and swirls along the curved inner surface. Then, the swirling flow of the physiological saline solution is led to the narrowing chamber 113, and is concentrated in the central axis direction of the swirling flow (axial centralized). Such a vortex is known as the Rankine vortex, and the inertia force of the swirling flow can be concentrated in the vicinity of the rotation axis of the vortex.

The jet flow of the contrast agent collides with the swirling flow of the physiological saline solution in the swirling flow generating chamber 112. Then, the swirling flow begins to form swirl motion so as to suck in the jet flow from the circumference. Thereafter, the peak of the swirl intensity of the swirling flow is shifted to be close to the central axis of the mixing device 110 from the outer periphery side of the swirling flow generating chamber 112 toward the tip of the narrowing chamber 113. Then, the jet flow is guided to and sucked in the vortex center of the swirling flow to which the swirl intensity is focused in this way.

According to this mixing device 110, a strong torque is applied to the contrast agent, which is a high viscosity fluid, and a centrifugal force is generated. As a result, the contrast agent is dispersed to the outer periphery direction of the swirling flow generating chamber 112. This flow structure is continuously formed in an injection process, and as a result, makes the entire flow field in the mixing device 110 to be turbulent. That is, the mixed liquid medicine of the contrast agent and the physiological saline solution is guided into the narrowing chamber 113, which is continuously narrowed toward the outlet 119, and thus the two fluids having different vectors positively collide to each other. In this manner, the contrast agent can be sucked in the vortex center, and can be made to continuously disperse to the outer periphery direction of the swirling flow generating chamber 112. As a result, the entire flow field is made to be turbulent, and the contrast agent and the physiological saline solution are efficiently mixed together.

Thereafter, the flow of the mixed liquid medicine of the contrast agent and the physiological saline solution is rectified in the narrowing chamber 113. Then, the rectified mixed liquid medicine flows out of the conduit portion 116 into the tube 144 through the outlet 119.

### [Air Venting]

Subsequently, air venting of the mixing tube 140 is described. First, the user takes out the mixing tube 140 from a sterilization bag, and removes the caps 146. Then, the user connects the first syringe 131 filled with the contrast agent to the terminal end portion 152 of the holding portion 150. Similarly, the user connects the second syringe 132 filled with the physiological salt solution to the unidirectional valve 141. Next, the user performs priming for the purpose of air venting. When manually performing priming, the user turns the respective pistons of the first syringe 131 and the second syringe 132 downward in the gravity direction. Then, the swirling flow generating chamber 112 is held by the holding portion 150 having a rigidity so that the conduit portion 116 is oriented upward in the gravity direction.

On this occasion, since the tube 144 is bent with its own flexibility, the male rotary lock connector 145 is located lower than the mixing device 110 in the gravity direction. Thereafter, the user first presses the piston of the first syringe 131 to push out the contrast agent, and fills the inside of the holding portion 150 (the internal flow passage 153) with the contrast agent. Next, the piston of the second syringe 132 is pressed to push out the physiological saline solution, and the inside of the mixing device 110 is filled with the physiological saline solution. In this manner, the mixing tube 140 is filled with the liquid medicine. An excessive liquid medicine is discharged from the male rotary lock connector 145, and collected in the liquid reservoir cap 147.

As shown in FIG. 4, an air bubble B tends to remain in the corner on the side of the jointing portion 160 of the swirling flow generating chamber 112. However, according to the holding portion 150 of the first embodiment, the swirling flow generating chamber 112 can be held so that the jointing portion 160 side is located lower in the gravity direction at the time of air venting. Therefore, since the liquid medicine also flows into the corner on the side of the jointing portion 160 with gravity, the air bubble B can be washed away to be discharged out of the mixing device 110 through the outlet 119. Further, the air bubble B can be washed away only by making a small amount of a liquid medicine flow in, or only by making the physiological saline solution flow in.

Thereafter, the user visually looks for an air bubble in the mixing tube 140, and when the user determines that air venting is completed, the user removes the liquid reservoir cap 147. Then, the user mounts the first syringe 131 and the second syringe 132 to the injection apparatus 120. Subsequently, the user connects the male rotary lock connector 145 to a catheter for contrast or an indwelling needle indwelled in a subject's body, and performs injection and imaging of the liquid medicine. Further, at the time of air venting, the contrast agent may be made to flow in the mixing device 110 after pushing out the physiological saline solution first, or the physiological saline solution and the contrast agent may be made to simultaneously flow in the mixing device 110.

### [Injection of Liquid Medicine]

Next, the injection of a liquid medicine is described. First, after mounting the first syringe 131 and the second syringe 132 to the injection apparatus 120, the user turns on the power supply of the injection apparatus 120. Then, the user operates the operation buttons of the injection apparatus 120, or the operation buttons displayed on the touch panel of control apparatus, and inputs, to the control apparatus, data required for creating an injection protocol. For example, the required data is physical data of a subject, such as the weight, the height, the surface area of a body, the heart rate, and the cardiac output, and data of the kinds of liquid medicines, etc. Further, the user may turn on the power supply of the injection apparatus 120 before mounting the syringes. Additionally, the data of the injection protocol and the liquid medicine can also be input to the control apparatus from an external storage medium.

The control apparatus stores in advance basic injection protocols, such as the injection speed, the injection rate, the injection time, and the injection maximum pressure (injection pressure limit value), and the data of liquid medicines. Then, the control apparatus determines an individual injection protocol suitable for an individual subject according to the input data and the data stored in advance. Additionally, the control apparatus displays predetermined data, such as the injection speed, the injection amount, and the injection time, on a touch panel. The user confirms the contents of the determined injection protocol, and changes the contents when necessary. Further, the data of the injection protocol and the liquid medicine can also be input to the control apparatus from an external apparatus, such as an imaging apparatus. Additionally, the injection protocol may be locked with a password, so that a third party cannot change the injection protocol.

Additionally, the control apparatus may display the injection protocol on external devices, such as a portable display or a tablet computer. These devices are wirelessly connected to an injection head of the injection apparatus 120 or the control apparatus in accordance with standards, such as Bluetooth (registered trademark) or Wi-Fi, and can be used as a head display of the injection head.

When the injection protocol is confirmed, the user presses the check button from among the operation buttons. In this manner, the injection apparatus 120 stands by in an injectable state. Thereafter, the user starts injection by pressing the start button of the operation buttons, or the start button of the remote operation apparatus. Thereafter, the injection apparatus 120 automatically injects the liquid medicine according to the injection protocol. Further, when the injection apparatus 120 includes a head display, the user can also start injection by pressing the start button displayed on the head display.

According to the first embodiment described above, when the user manually vents the air, it is possible to suppress that air bubbles remain in the mixing device, and to vent the air with a small amount of liquid medicine. Additionally, according to the mixing device 110, it is possible to make the contrast agent turbulent, which has a high specific gravity and is a high viscosity fluid, and to efficiently perform mixing in the three dimensional directions in the mixing device 110. As a result, mixing efficiency can be significantly improved.

Additionally, according to the mixing device 110, even with a little amount of contrast agent and physiological saline solution, vortex can be generated in tens of milliseconds. Therefore, it is possible to mix the contrast agent with the physiological saline solution in a small amount of time, and to reliably mix a little amount of contrast agent with the physiological saline solution. As a result, the effect that generation of unevenness of an image can be suppressed is achieved.

Further, instead of manual air venting, the air can also be vented automatically or according to a user's operation by using the injection apparatus 120. Specifically, the first syringe 131 and the second syringe 132 are mounted to the injection apparatus 120, and the rear end side of the injection head 121 is oriented to the floor surface. Next, the mixing tube 140 is connected to the first syringe 131 and the second syringe 132. Then, the air can be vented by pushing out the liquid medicine while maintaining the injection head 121 perpendicular in the gravity direction. Additionally, the liquid medicines to be mixed are not limited to the contrast agent and the physiological saline solution. For example, one of two kinds of liquids may be a mixed liquid medicine of the contrast agent and the physiological saline solution, and the other may be the physiological saline solution. In this case, the first syringe 131 is filled with the mixed liquid medicine (the contrast agent and the physiological saline solution) having a specific gravity and a viscosity higher than those of the physiological saline solution. Then, the second syringe 132 is filled with the physiological saline solution.

### [Second Embodiment]

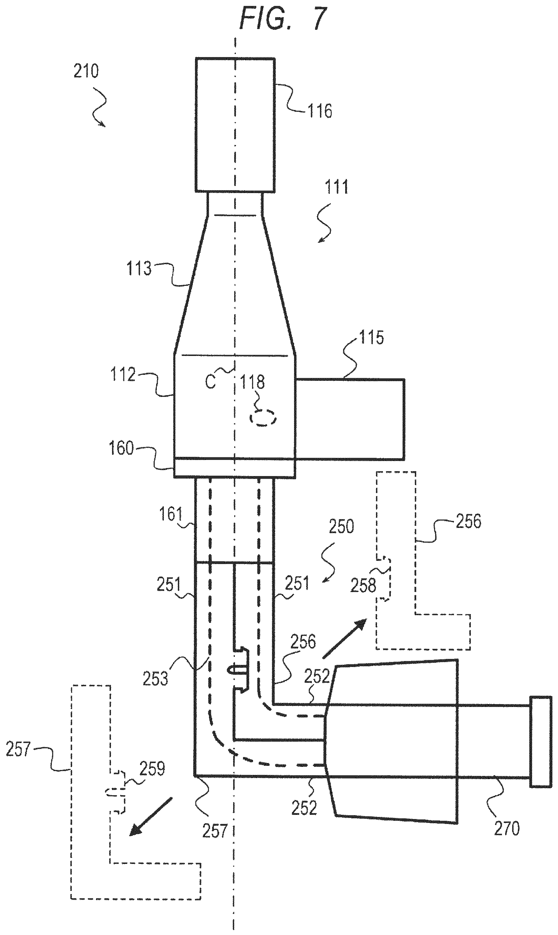

A second embodiment is described with reference to FIG. 7. FIG. 7 is a schematic side view along the longitudinal direction of the mixing device 210. Further, in FIG. 7, for convenience of description, the inner part 256 and the outer part 257 of the removed holding portion 250 are shown by dotted lines. In the description of the second embodiment, the difference with the first embodiment is described, and a description about the components described in the first embodiment will be omitted. Unless specifically described, components with the same reference numerals achieve the substantially same operation and function, and their effects are also substantially the same.

A mixing device 210 according to the second embodiment also includes the swirling flow generating chamber 112, the narrowing chamber 113, the conduit portion 115, the conduit portion 116, and the jointing portion 160. Then, when injecting a mixed liquid medicine, a liquid medicine having a large specific gravity from the first syringe 131 (the contrast agent) is made to flow into the swirling flow generating chamber 112. Additionally, a liquid medicine having a small specific gravity from the second syringe 132 (the physiological saline solution) is made to flow into the swirling flow generating chamber 112. Thereafter, the flow of the mixed liquid medicine of the contrast agent and the physiological saline solution is rectified in the narrowing chamber 113. Then, the rectified mixed liquid medicine flows out of the conduit portion 116 into the tube 144.

The mixing device 210 of the second embodiment includes a removable holding portion 250 as a holding portion 250 that holds the swirling flow generating chamber 112. This holding portion 250 includes a rigid inner part (a first part) 256 and an outer part (a second portion) 257 separable from each other, and a flexible flow passage tube 253 arranged between the inner part 256 and the outer part 257. Each of the inner part 256 and the outer part 257 has a substantially L shape. Additionally, the inner part 256 and the outer part 257 have a groove portion (not shown) with a substantially semicircular cross section. Additionally, a flow passage tube 253 is arranged in the groove portion.

The inner part 256 has a female engagement portion (a first engagement portion) 258, and the outer part 257 has a male engagement portion (a second engagement portion) 259 to be engaged with the female engagement portion 258. Additionally, when the inner part 256 and the outer part 257 are combined, a convex portion of the male engagement portion 259 is inserted into a concave portion of the female engagement portion 258, and both of them are fixed by snap fit. When removing the holding portion 250, the male engagement portion 259 is pressed toward the inside (the flow passage tube 253 side) to be deformed, and the convex portion of the male engagement portion 259 is separated from the concave portion of the female engagement portion 258. In this manner, since the engagement is canceled, the inner part 256 and the outer part 257 can be removed in the directions indicated by the arrows of FIG. 7.

Additionally, the holding portion 250 includes a tip portion 251 extending in a direction parallel to a center line C of the swirling flow generating chamber 112. That is, a part of the inner part 256 and the outer part 257 engaged with each other forms the tip portion 251. Additionally, the other part of the inner part 256 and the outer part 257 engaged with each other forms a terminal end portion 252 that is bent and extends from the tip portion 251. This terminal end portion 252 extends in a direction substantially orthogonal to the extending direction of the tip portion 251 (the center line C of the swirling flow generating chamber 112).

The length of the flow passage tube 253 is set to an extent that does not to protrude from between the inner part 256 and the outer part 257 when both of them are combined. In this manner, when both of them are combined, the posture of the flow passage tube 253 becomes a substantially L shape according to the shapes of the inner part 256 and the outer part 257. A flow passage with a substantially circular cross-section is formed inside of this flow passage tube 253 so that the liquid medicine (the contrast agent) flows. Further, the holding portion 250 can be formed by molding resin, such as polycarbonate, for example. Additionally, the flow passage tube 253 can be formed from polyvinyl chloride, for example.

The swirling flow generating chamber 112 includes the jointing portion 160 to be joined to the flow passage tube 253. This jointing portion 160 includes the substantially cylindrical receiving portion 161 for receiving a downstream side end portion of the flow passage tube 253. Then, the downstream side end portion of the flow passage tube 253 in the flowing direction of the liquid medicine is joined to the receiving portion 161 of the jointing portion 160. Additionally, an upstream side end portion of the flow passage tube 253 in the flowing direction of the liquid medicine is joined to a female connector 270 to be connected to the second syringe 132.

The downstream side end portion of the flow passage tube 253 inserted in the jointing portion 160 communicates with the swirling flow generating chamber 112 via the first inlet 117. In this manner, the liquid medicine having a large specific gravity that passed the first inlet 117 is introduced into the swirling flow generating chamber 112 in a direction parallel to the central axis of the swirling flow of the liquid medicine having a small specific gravity. For example, the downstream side end portion and the upstream side end portion can be bonded by solvent adhesion, respectively.

### [Air Venting]

Subsequently, though air venting of the liquid medicine is described, since the process until the first syringe 131 and the second syringe 132 are connected is similar to that of the first embodiment, a description of the process is omitted. When the first syringe 131 and the second syringe 132 are connected, the user orients each of the first syringe 131 and the second syringe 132 to be horizontal to a ground surface. Then, the swirling flow generating chamber 112 is held by the holding portion 250 having a rigidity so that the conduit portion 116 is oriented upward in the gravity direction.

On this occasion, since the tube 144 is bent with its own flexibility, the male rotary lock connector 145 is located lower than the mixing device 210 in the gravity direction. Thereafter, the user first presses the piston of the first syringe 131 to push out the contrast agent, and the inside of the flow passage tube 253 and the female connector 270 is filled with the contrast agent. Next, the piston of the second syringe 132 is pressed to push out the physiological saline solution, and the inside of the mixing device 210 is filled with the physiological saline solution. In this manner, the mixing tube 140 is filled with the liquid medicine. An excessive liquid medicine is discharged from the male rotary lock connector 145, and is collected in the liquid reservoir cap 147.

The swirling flow generating chamber 112 can be held so that the jointing portion 160 side is located lower in the gravity direction also by the holding portion 250 of the second embodiment at the time of air venting. Therefore, since the liquid medicine also flows into the corner on the side of the jointing portion 160 with gravity, air bubbles can be washed away to be discharged out of the mixing device 210. Further, the user can perform air venting in the state where the first syringe 131 and the second syringe 132 are horizontal to the ground surface. In this manner, since it becomes easy for the user to push the piston, air venting can be easily performed.

Thereafter, the user visually looks for an air bubble in the mixing tube 140, and when the user determines that air venting is completed, the user removes the liquid reservoir cap 147. Then, the user mounts the first syringe 131 and the second syringe 132 to the injection apparatus 120. Subsequently, the user connects the male rotary lock connector 145 to a catheter for contrast or an indwelling needle indwelled in a subject's body, and performs injection and imaging of the liquid medicine. Further, at the time of air venting, the contrast agent may be made to flow in the mixing device 210 after pushing out the physiological saline solution first, or the physiological saline solution and the contrast agent may be made to simultaneously flow in the mixing device 210.

According to the second embodiment described above, when the user manually vents the air, it is possible to suppress that air bubbles remain in the mixing device, and to vent the air with a small amount of liquid medicine. Further, according to the second embodiment, air venting can be performed without standing the first syringe 131 and the second syringe 132. Further, with the holding portion 250, the mixing device 210 can also be arranged so that the outlet 119 is located lower than the first inlet 117 in the gravity direction. That is, it is possible to change the orientations and switch the positions of the inner part 256 and the outer part 257 shown in FIG. 7, so that the combined holding portion 250 is bent toward the floor surface. In this manner, the central axis lines of the first inlet 117, the swirling flow generating chamber 112, and the narrowing chamber 113 can be set to be parallel to the gravity direction. Therefore, the axial symmetry property of the system of the mixed liquid medicine in the mixing device 210 can be increased, and liquid medicines can be mixed more effectively.

According to the mixing device 210, it is possible to make the contrast agent turbulent, which has a large specific gravity and is a high viscosity fluid, and to efficiently perform mixing in the three dimensional directions in the mixing device 110. As a result, mixing efficiency can be significantly improved. Additionally, according to the mixing device 210, even with a little amount of contrast agent and physiological saline solution, vortex can be generated in tens of milliseconds. Therefore, it is possible to mix the contrast agent with the physiological saline solution in a small amount of time, and to reliably mix a little amount of contrast agent with the physiological saline solution. As a result, the effect that generation of unevenness of an image can be suppressed is achieved.

### [Modification]

Although the present invention has been described above with reference to each of the embodiments, the present invention is not limited to the above-described embodiments. An invention changed within a scope that does not contradict to the present invention, and an invention equivalent to the present invention are also included in the present invention. Additionally, each of the embodiments and each of the modifications can be properly combined within a scope that not contradict to the present invention.

For example, a taper portion can be formed between the inner surface of the swirling flow generating chamber 112 and the inner end surface on the side of the first inlet 117 of the swirling flow generating chamber 112. Additionally, a gentle curved surface can also be provided in the boundary part of the swirling flow generating chamber 112 and the narrowing chamber 113, so that the inner surface of the narrowing chamber 113 has a streamline shape. In this manner, it becomes difficult for air bubbles to adhere to the inner surface of the mixing device 110, and friction can be made small.

Further, a gentle curved surface can also be formed in an outside surface of the mixing device 110, especially, the boundary of the swirling flow generating chamber 112 and the narrowing chamber 113. Additionally, the swirling flow generating chamber 112, the narrowing chamber 113, and the conduit portion 116 can be configured to have a common substantially cylindrical outer shape, so that the swirling flow generating chamber 112, the narrowing chamber 113, and the conduit portion 116 have a common curved side surface. In this manner, in the case of manufacturing with a metal mold, the shape of the metal mold can be simplified. Additionally, in the case of manufacturing by shaving a base material, the amount of shaving can be reduced.

Additionally, the swirling flow generating chamber 112 can also be configured to have a narrowed shape that narrows toward the narrowing chamber 113. For example, the inner surface of the swirling flow generating chamber 112 and the inner surface of the narrowing chamber 113 can be formed by one surface which inclines by the same inclination with respect to the central axis line of the conical space which the swirling flow generating chamber 112 has. Additionally, the narrowed shape of the swirling flow generating chamber 112 can be configured so that the inclination of the inner surface of the narrowing chamber 113 with respect to the central axis line of the swirling flow generating chamber 112 becomes larger than the inclination of the inner surface of the swirling flow generating chamber 112 with respect to the central axis line of the swirling flow generating chamber 112.

Additionally, the holding portion 150, the swirling flow generating chamber 112, the narrowing chamber 113, the jointing portion 160, the conduit portion 115, and the conduit portion 116 may be integrally molded by the same member. Further, each of the swirling flow generating chamber 112, the narrowing chamber 113, the conduit portion 115, and the conduit portion 116 may be formed with another body, and an integral mixing device 110 may be formed by bonding them to each other.

Additionally, the inner diameter of the outlet 119 can also be made larger than the first inlet 117. Further, in the first and second embodiments, the length of the swirling flow generating chamber 112 along the center line C was shorter than the length of the narrowing chamber 113. However, in order to secure a sufficient capacity, the swirling flow generating chamber 112 can be made longer than the narrowing chamber 113.

Additionally, hydrophilizing processing can be performed on the inner surface of the mixing device 110. By performing the hydrophilizing processing, it is possible to prevent an air bubble from adhering to the mixing device 110 inner surface when performing air venting. As the methods for this hydrophilizing processing, there are plasma processing, ozonation processing, corona discharge processing, glow-discharge processing, and ultraviolet light irradiation processing.

### Reference Signs List

100: injection system, 110: mixing device, 112: swirling flow generating chamber, 113: narrowing chamber, 117: first inlet, 118: second inlet, 119: outlet, 120: injection apparatus, 131: first syringe, 132: second syringe, 140: mixing tube, 143: tube, 144: tube, 150: holding portion, 151: tip portion, 160: jointing portion, 161: receiving portion, 162: adhesive reservoir, 210: mixing device, 250: holding portion, 251: tip portion, 252: terminal end portion, 253: flow passage tube, 250: first part, 257: second part, 258: first engagement portion, 259: second engagement portion, A: adhesive

## Claims

1. A mixing device (110, 210) comprising:
a swirling flow generating chamber (112) configured to generate a swirling flow;
a first inlet (117) configured to introduce a first liquid medicine into the swirling flow generating chamber (112);
a second inlet (118) configured to introduce a second liquid medicine into the swirling flow generating chamber (112);
an outlet (119) from which a mixed liquid medicine of the first liquid medicine and the second liquid medicine flows out;
a narrowing chamber (113) provided between the swirling flow generating chamber (112) and the outlet (119), and includes a space continuously narrowed toward the outlet (119); **characterized by:**
a rigid holding portion (150, 250) configured to include a tip portion (151, 251) extending in a direction parallel to a center line of the swirling flow generating chamber (112), wherein the holding portion (150, 250) includes an internal flow passage (153) and a terminal end portion (152) and has a tapered shape in which the cross-sectional area is decreased from the terminal end portion (152) to the tip portion (151), wherein the internal flow passage (153) is formed to be narrowed toward the first inlet (117).

2. A mixing device (110, 210) according to claim 1, wherein the swirling flow generating chamber (112) includes a jointing portion (160) to joint the tip portion (151, 251),
inserted into a receiving portion (161) of the jointing portion (160).

3. A mixing device (110, 210) according to claim 2, wherein the tip portion (151, 251) is smaller than the receiving portion (161) and has a tapered shape, and
an adhesive reservoir (162) is provided between the tip portion (151, 251) and the receiving portion (161).

4. A mixing device (110, 210) according to any one of claims 1 to 3, wherein the holding portion (150, 250) includes a terminal end portion (252) for connecting a syringe filled with the first liquid medicine.

5. A mixing device (110, 210) according to claim 1, wherein the holding portion (150, 250) includes a first part (250) and a second part (256) separable from each other, and a flow passage tube (253) arranged between the first part (250) and the second part (257),
the first part (256) includes a first engagement portion (258), and
the second part (257) includes a second engagement portion (259) to be engaged with the first engagement portion (258).

6. A mixing device (110, 210) according to claim 5, wherein the swirling flow generating chamber (112) includes a jointing portion (160) to joint the flow passage tube (253), and
the first part (256) and the second part (257) engaged with each other form the tip portion (151, 251) and a terminal end portion (252) that is bent and extends from the tip portion (151, 251).

7. A mixing tube (140) comprising:
a tube (143) configured to communicate with the second inlet (118);
a tube (144) configured to communicate with the outlet (119); and
the mixing device (110, 210) according to any one of claims 1 to 6.

8. An injection system (100) comprising:
an injection apparatus (120) to which a first syringe (131) filled with the first liquid medicine and a second syringe (132) filled with the second liquid medicine are mounted; and
the mixing tube (140) according to claim 7 connected to the first syringe (131) and the second syringe (132).

9. A mixing device (110, 210) manufacturing method comprising:
forming a swirling flow generating chamber (112) to have a jointing portion (160) including a receiving portion (161), **characterized by** forming a rigid holding portion (150, 250) to have a tip portion (151, 251) smaller than the receiving portion (161), wherein the holding portion (150, 250) includes an internal flow passage (153) and a terminal end portion (152) and has a tapered shape in which the cross-sectional area is decreased from the terminal end portion (152) to the tip portion (151);
applying an adhesive (A) between the tip portion (151, 251) and the receiving portion (161); and
curing the adhesive (A).

10. A mixing device (110, 210) manufacturing method according to claim 9, comprising:
forming the holding portion (150, 250) so that the tip portion (151, 251) has a tapered shape; and
in a case where the adhesive (A) is applied, applying the adhesive (A) to the tip portion (151, 251), and inserting the tip portion (151, 251) to which the adhesive (A) is applied into the receiving portion (161).

## Patentansprüche

1. Eine Mischvorrichtung (110, 210), umfassend:
eine Wirbelstromerzeugungskammer (112), die konfiguriert ist, einen Wirbelstrom zu erzeugen;
einen ersten Einlass (117), der konfiguriert ist, ein erstes flüssiges Medikament in die Wirbelstromerzeugungskammer (112) einzuleiten;
einen zweiten Einlass (118), der konfiguriert ist, ein zweites flüssiges Medikament in die Wirbelstromerzeugungskammer (112) einzuleiten;
einen Auslass (119), aus dem ein gemischtes flüssiges Medikament aus dem ersten flüssigen Medikament und dem zweiten flüssigen Medikament austritt;
eine Verengungskammer (113), die zwischen der Wirbelstromerzeugungskammer (112) und dem Auslass (119) angeordnet ist und einen Raum umfasst, der sich zum Auslass (119) hin kontinuierlich verengt; **gekennzeichnet durch:**
einen starren Halteabschnitt (150, 250), der konfiguriert ist, einen Spitzenabschnitt (151, 251) zu umfassen, der sich in einer Richtung parallel zu einer Mittellinie der Wirbelstromerzeugungskammer (112) erstreckt, wobei der Halteabschnitt (150, 250) einen inneren Strömungskanal (153) und einen Spitzenendabschnitt (152) aufweist und eine sich verjüngende Form hat, bei der sich die Querschnittsfläche vom Endabschnitt (152) zum Spitzenabschnitt (151) hin verringert, wobei der innere Strömungskanal (153) ausgebildet ist, sich zum ersten Einlass (117) hin zu verjüngen.

2. Die Mischvorrichtung (110, 210) gemäß Anspruch 1, wobei die Wirbelstromerzeugungskammer (112) einen Verbindungsabschnitt (160) zum Verbinden des Spitzenabschnitts (151, 251) umfasst, der in einen Aufnahmebereich (161) des Verbindungsabschnitts (160) eingeführt ist.

3. Die Mischvorrichtung (110, 210) gemäß Anspruch 2, wobei der Spitzenabschnitt (151, 251) kleiner als der Aufnahmeabschnitt (161) ist und eine konische Form aufweist, und
ein Klebstoffreservoir (162) zwischen dem Spitzenabschnitt (151, 251) und dem Aufnahmeabschnitt (161) angeordnet ist.

4. Die Mischvorrichtung (110, 210) gemäß einem der Ansprüche 1 bis 3, wobei der Halteabschnitt (150, 250) einen Anschlussendabschnitt (252) zur Verbindung mit einer mit dem ersten flüssigen Medikament gefüllten Spritze umfasst.

5. Die Mischvorrichtung (110, 210) gemäß Anspruch 1, wobei der Halteabschnitt (150, 250) einen ersten Teil (250) und einen zweiten Teil (256) umfasst, die voneinander trennbar sind, sowie ein Strömungskanalrohr (253), das zwischen dem ersten Teil (250) und dem zweiten Teil (257) angeordnet ist,
der erste Teil (256) einen ersten Eingriffsabschnitt (258) umfasst, und
der zweite Teil (257) einen zweiten Eingriffsabschnitt (259) umfasst, der mit dem ersten Eingriffsabschnitt (258) in Eingriff gebracht wird.

6. Die Mischvorrichtung (110, 210) gemäß Anspruch 5, wobei die Wirbelstromerzeugungskammer (112) einen Verbindungsabschnitt (160) zum Verbinden des Strömungskanalrohrs (253) umfasst, und
der erste Teil (256) und der zweite Teil (257), die miteinander in Eingriff stehen, den Spitzenabschnitt (151, 251) und einen Endabschnitt (252) bilden, der gebogen ist und sich vom Spitzenabschnitt (151, 251) erstreckt.

7. Ein Mischrohr (140), umfassend:
einem Rohr (143), das konfiguriert ist, mit dem zweiten Einlass (118) in Verbindung zu stehen;
ein Rohr (144), das konfiguriert ist, mit dem Auslass (119) in Verbindung zu stehen; und
die Mischvorrichtung (110, 210) gemäß einem der Ansprüche 1 bis 6.

8. Ein Injektionssystem (100), umfassend:
eine Injektionsvorrichtung (120), an der eine mit dem ersten flüssigen Medikament gefüllte erste Spritze (131) und eine mit dem zweiten flüssigen Medikament gefüllte zweite Spritze (132) angebracht sind; und
das Mischrohr (140) gemäß Anspruch 7, das mit der ersten Spritze (131) und der zweiten Spritze (132) verbunden ist.

9. Ein Verfahren zur Herstellung einer Mischvorrichtung (110, 210), umfassend:
das Ausbilden einer Wirbelstromerzeugungskammer (112) mit einem Verbindungsabschnitt (160), der einen Aufnahmeabschnitt (161) umfasst, **gekennzeichnet durch** das Ausbilden eines starren Halteabschnitts (150, 250) mit einem Spitzenabschnitt (151, 251) aufweist, der kleiner ist als der Aufnahmebereich (161), wobei der Halteabschnitt (150, 250) einen inneren Strömungskanal (153) und einen Endabschnitt (152) umfasst und eine konische Form aufweist, bei der sich die Querschnittsfläche vom Endabschnitt (152) zum Spitzenabschnitt (151) hin verjüngt;
Aufbringen eines Klebstoffs (A) zwischen dem Spitzenabschnitt (151, 251) und dem Aufnahmeabschnitt (161); und
Aushärten des Klebstoffs (A).

10. Ein Verfahren zur Herstellung einer Mischvorrichtung (110, 210) gemäß Anspruch 9, umfassend:
Ausbilden des Halteabschnitts (150, 250) derart, dass der Spitzenabschnitt (151, 251) eine sich verjüngende Form aufweist; und
in einem Fall, in dem der Klebstoff (A) aufgetragen wird, das Auftragen des Klebstoffs (A) auf den Spitzenabschnitt (151, 251) und das Einführen des Spitzenabschnitts (151, 251), auf den der Klebstoff (A) aufgetragen wurde, in den Aufnahmeabschnitt (161).

## Revendications

1. Dispositif mélangeur (110, 210) comprenant :
une chambre de génération d'écoulement tourbillonnaire (112) configurée pour générer un écoulement tourbillonnaire ;
une première entrée (117) configurée pour introduire un premier médicament liquide dans la chambre de génération d'écoulement tourbillonnaire (112) ;
une deuxième entrée (118) configurée pour introduire un deuxième médicament liquide dans la chambre de génération d'écoulement tourbillonnaire (112) ;
une sortie (119) d'où s'écoule un médicament liquide mélangé du premier médicament liquide et du deuxième médicament liquide ;
une chambre de rétrécissement (113) pourvue entre la chambre de génération d'écoulement tourbillonnaire (112) et la sortie (119), et incluant un espace qui se rétrécit continuellement vers la sortie (119) ;
**caractérisé par** :
une portion de maintien rigide (150, 250) configurée pour inclure une portion de pointe (151, 251) qui s'étend dans une direction parallèle à une ligne centrale de la chambre de génération d'écoulement tourbillonnaire (112), dans lequel la portion de maintien (150, 250) inclut un passage d'écoulement interne (153) et une portion d'extrémité terminale (152), et présente une forme conique où la section transversale diminue depuis la portion d'extrémité terminale (152) vers la portion de pointe (151), dans lequel le passage d'écoulement interne (153) est formé pour se rétrécir vers la première entrée (117).

2. Dispositif mélangeur (110, 210) selon la revendication 1, dans lequel la chambre de génération d'écoulement tourbillonnaire (112) inclut une portion de jonction (160) pour joindre la portion de pointe (151, 251), insérée dans une portion de réception (161) de la portion de jonction (160).

3. Dispositif mélangeur (110, 210) selon la revendication 2, dans lequel la portion de pointe (151, 251) est plus petite que la portion de réception (161) et présente une forme conique, et un réservoir d'adhésif (162) est pourvu entre la portion de pointe (151, 251) et la portion de réception (161).

4. Dispositif mélangeur (110, 210) selon l'une quelconque des revendications 1 à 3, dans lequel la portion de maintien (150, 250) inclut une portion d'extrémité terminale (252) pour connecter une seringue remplie du premier médicament liquide.

5. Dispositif mélangeur (110, 210) selon la revendication 1, dans lequel
la portion de maintien (150, 250) inclut une première partie (250) et une deuxième partie (256) séparables l'une de l'autre, et un tube de passage d'écoulement (253) agencé entre la première partie (250) et la deuxième partie (257),
la première partie (256) inclut une première portion d'engagement (258), et
la deuxième partie (257) inclut une deuxième portion d'engagement (259) à engager avec la première portion d'engagement (258).

6. Dispositif mélangeur (110, 210) selon la revendication 5, dans lequel
la chambre de génération d'écoulement tourbillonnaire (112) inclut une portion de jonction (160) pour joindre le tube de passage d'écoulement (253), et
la première partie (256) et la deuxième partie (257) engagées l'une avec l'autre forment la portion de pointe (151, 251) et une portion d'extrémité terminale (252) qui est pliée et s'étend depuis la portion de pointe (151, 251).

7. Tube mélangeur (140) comprenant :
un tube (143) configuré pour communiquer avec la deuxième entrée (118) ;
un tube (144) configuré pour communiquer avec la sortie (119) ; et
le dispositif mélangeur (110, 210) selon l'une quelconque des revendications 1 à 6.

8. Système d'injection (100) comprenant :
un appareil d'injection (120) sur lequel sont montées une première seringue (131) remplie du premier médicament liquide et une deuxième seringue (132) remplie du deuxième médicament liquide ; et
le tube mélangeur (140) selon la revendication 7, connecté à la première seringue (131) et à la deuxième seringue (132).

9. Procédé de fabrication d'un dispositif mélangeur (110, 210), comprenant :
la formation d'une chambre de génération d'écoulement tourbillonnaire (112) de manière à ce qu'elle comporte une portion de jonction (160) incluant une portion de réception (161),
**caractérisé par**
la formation d'une portion de maintien rigide (150, 250) de manière à ce qu'elle comporte une portion de pointe (151, 251) plus petite que la portion de réception (161), dans lequel la portion de maintien (150, 250) inclut un passage d'écoulement interne (153) et une portion d'extrémité terminale (152), et présente une forme conique où la section transversale diminue depuis la portion d'extrémité terminale (152) vers la portion de pointe (151) ;
l'application d'un adhésif (A) entre la portion de pointe (151, 251) et la portion de réception (161) ; et
la polymérisation de l'adhésif (A).

10. Procédé de fabrication d'un dispositif mélangeur (110, 210) selon la revendication 9, comprenant :
la formation de la portion de maintien (150, 250) de manière à ce que la portion de pointe (151, 251) présente une forme conique ; et
dans le cas où l'adhésif (A) est appliqué, l'application de l'adhésif (A) à la portion de pointe (151, 251), et l'insertion de la portion de pointe (151, 251) où l'adhésif (A) est appliqué dans la portion de réception (161).
